(19) **Europäisches Patentamt**

**European Patent Office**

**Office européen des brevets**

(11) Veröffentlichungsnummer: **0 434 604 A1**

(12) # EUROPÄISCHE PATENTANMELDUNG

(21) Anmeldenummer: 90810855.8

(22) Anmeldetag: 07.11.90

(51) Int. Cl.$^5$: **A61F 2/36**

(30) Priorität: 15.12.89 CH 4512/89

(43) Veröffentlichungstag der Anmeldung:
26.06.91 Patentblatt 91/26

(84) Benannte Vertragsstaaten:
AT CH DE FR GB IT LI

(71) Anmelder: GEBRÜDER SULZER
AKTIENGESELLSCHAFT
Zürcherstrasse 9
CH-8401 Winterthur (CH)

(72) Erfinder: Scherrer, Markus, Dr.-med.
Sarganserstrasse 23
CH-7310 Bad Ragaz (CH)
Erfinder: Koch, Rudolf
Oberdorfstrasse 229
CH-8267 Berlingen (CH)
Erfinder: Willert, Hans-Georg, Prof.Dr.-med.
Schlegelweg 9
W-3400 Göttingen (DE)

(54) **Verankerungsschaft für eine Femurkopfprothese.**

(57)    Der Schaft (3) einer Femurkopfprothese (5) ist in Richtung seiner Längsachse (9) mit einer möglichst, in dieser Achse (9) gelegenen, Bohrung (10) versehen, die über einen Durchbruch (14) mit der Aussenseite des Schaftes (3) und damit mit dem Operationshohlraum (2) in Verbindung steht.

Bohrung (10), auf deren proximales Ende druckdicht eine Knochenzementspritze (16) aufsetzbar ist, und Durchbruch (14) ermöglichen ein Einbringen des Knochenzementes (4) nach dem Einsetzen des Schaftes (3) in den Hohlraum (2), wodurch die neue Implantiertechnik, bei der der Knochenzement (4) erst nach dem Schaft (3) in den Femur (1) eingebracht wird, erheblich erleichtert wird.

FIG. 1

# VERANKERUNGSSCHAFT FÜR EINE FEMURKOPFPROTHESE

Die Erfindung betrifft einen Verankerungsschaft für eine Femurkopfprothese, der mit Hilfe eines Knochenzementbettes im Femur fixiert werden soll, wobei der Schaft in Richtung seiner zentralen Längsachse eine Bohrung aufweist, die am distalen Ende verschlossen ist.

Schäfte der genannten Art sind beispielsweise bekannt aus der EP-A-0 331 623, der WO 86/02260 und der DE-A-33 04 476 ; bei der genannten EP-A-0 331 623 und der DE-A-33 04 476 dient die Längsbohrung zur Aufnahme eines Führungselementes, mit dessen Hilfe der Schaft im Operationshohlraum "zentriert" werden soll. Sobald die Prothese in der richtigen Lage fixiert ist, werden die Führungselemente entfernt. Bei der Veröffentlichung WO 86/02260 dient die Bohrung zur Aufnahme eines Zugankers, mit dem ein der Fixierung dienendes aufspreizbares Element am distalen Ende des Schaftes aufgespreizt wird.

In letzter Zeit ist man bei Implantationen, bei denen der Schaft mit Hilfe eines Knochenzementbettes im Knochen fixiert wird, dazu übergegangen, den Schaft in den Operationshohlraum einzusetzen, ehe der Knochenzement eingefüllt wird.

Aufgabe der Erfindung ist es, diese neue Zementiertechnik für die Schaftkonstruktionen mit einer Längsbohrung zu vereinfachen.

Diese Aufgabe wird mit der Erfindung dadurch gelöst, dass die Bohrung über mindestens einen Durchbruch im distalen Bereich mit der Aussenseite des Schaftes in Verbindung steht.

Beispielsweise kann ein Schaft, bei dem vorteilhafterweise die Bohrung seine ganze Länge durchsetzt und am distalen Ende durch eine eingesetzte Markraumsperre verschlossen ist, mit der neuen Erfindung in den leeren Knochenhohlraum eingesetzt und mit Hilfe der Markraumsperre distal zentriert werden. Anschliessend wird an das proximale Ende der Bohrung eine sogenannte Hochdruck-Zementspritze angesetzt, mit der der Zement durch die Bohrung und den Durchbruch mit Hilfe eines erhöhten Druckes in den Zwischenraum zwischen dem Prothesenschaft und der Wand des Operationshohlraumes eingepresst wird. Die Füllung des Hohlraumes erfolgt dabei von distal nach proximal ; dies bringt den zusätzlichen Vorteil, dass sehr einfach beobachtet werden kann, wann der Hohlraum gefüllt ist.

Aus Festigkeitsgründen ist es vorteilhaft, wenn der Durchbruch nach ventral/dorsal gerichtet ist ; weiterhin ist es zweckmässig, wenn die Bohrung in bekannter Weise in der Längsmittelachse des Schaftes angeordnet ist, da dort bei Torsions- und Biegebelastungen eine "neutrale" Zone vorhanden ist.

Da — wie geschildert — der Zement unter Druck eingepresst wird, ist es weiterhin vorteilhaft, wenn das proximale Ende der Längsbohrung zu einer Einsatzöffnung für ein mindestens näherungsweise druckdichtes Einsetzen einer Knochenzementspritze ausgeformt ist. Dafür hat es sich bewährt, wenn die Einsatzöffnung ein Gewinde zum Einschrauben der Spritze trägt, in das — nach Lösen der Spritze — zusätzlich als Verschlussdeckel eine Schraube einschraubbar ist.

Ferner ist es möglich, den Zementdruck zusätzlich durch eine bekannte Abdeckung an der Resektionsebene zu erhöhen, wobei diese Abdeckung mit einer Entlüftungsbohrung versehen sein kann.

Im folgenden wird die Erfindung anhand von Ausführungsbeispielen im Zusammenhang mit der Zeichnung näher erläutert.

Fig. 1 zeigt schematisch einen Längsschnitt durch eine Femurknochen, in den bei eingesetzter Femurkopfprothese Knochenzement eingefüllt wird ;

Fig. 2 ist ein Schnitt II-II von Fig. 1 ;

Fig. 3 und 4 geben in gleicher Darstellung eine zweite Ausführungsform der Erfindung wieder.

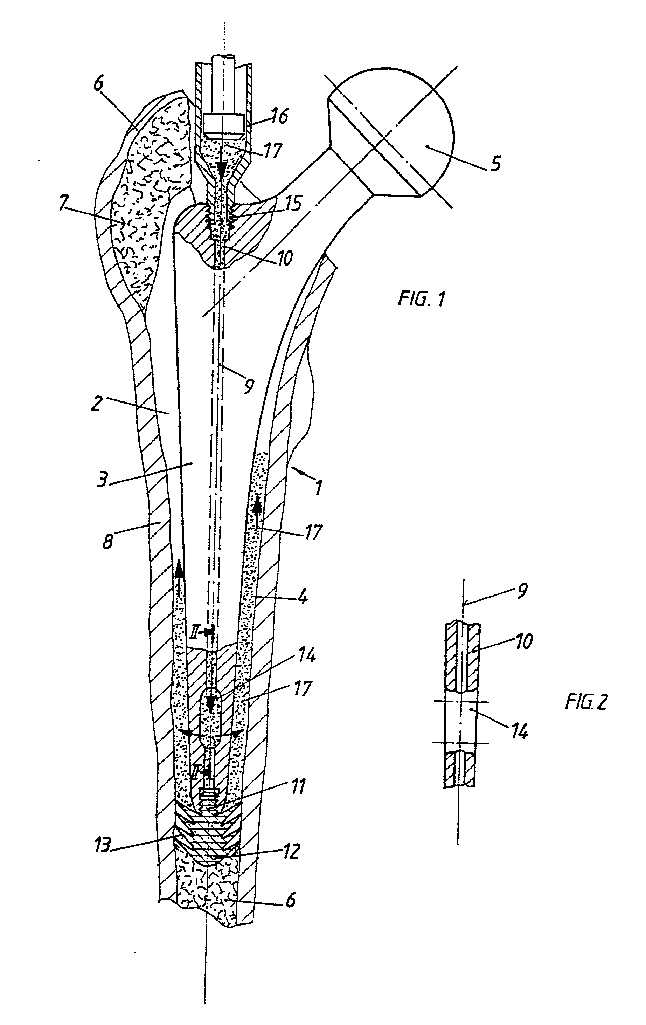

In dem durch einen Schnitt etwa senkrecht zur Schenkelhalsachse eröffneten Femur 1 (Fig. 1) ist ein Operationshohlraum 2 geschaffen worden, der einen Verankerungsschaft 3 einer Femurkopfprothese 5 aufnimmt ; dieser wird seinerseits mit Hilfe eines Knochenzementbettes 4 fixiert.

Ausser im Bereich des grossen Trochanters 6 ist die Spongiosa 7 mit einem raspelartigen Instrument ausgeräumt. Die Ausräumung wird durch den Rand der Kortikalis 8 begrenzt.

Der den gezeigten Beispielen als Geradschaft ausgeführte Schaft 3 ist in seiner Längsachse 9 mit einer Bohrung 10 versehen, die seine ganze Länge durchsetzt und am distalen Ende mit einem Gewinde 11 versehen ist. In dieses ist als distaler "Verschluss" der Bohrung 10 eine z.B. aus Kunststoff bestehende Markraumsperre 12 eingeschraubt. Neben ihrer Funktion, die Bohrung 10 zu verschliessen, hat die Markraumsperre 12 zum einen den Zweck, den Operationshohlraum 2 abzuschliessen und damit den Knochenzement 4 an einem Eindringen in das distal von Operationshohlraum 2 gelegene Knochenmarkgewebe 6 zu hindern. Zum anderen dient die Markraumsperre 12 dazu, den Schaft 3 beim Implantieren in dem Hohlraum 2 zu zentrieren, weshalb sie an ihrem Umfang mit elastischen Lappen 13 versehen ist.

Ueber einen Durchbruch 14, der den Schaft 3 in Richtung anterior/posterior (Fig. 2) durchsetzt, steht die Bohrung 10 mit der Aussenseite des Schaftes 3 in Verbindung. Das obere Ende der Bohrung 10 enthält ebenfalls ein Gewinde 15, in das ein Zylinder 16 einer Zementspritze druckdicht eingeschraubt ist. Wie durch Pfeile 17 angedeutet, wird nach dem Einsetzen des Schaftes 3 in den Hohlraum 2 der Knochenzement 4 aus dem Zylinder 16 durch die Bohrung 10 und den Durchbruch 14 in den Operationshohlraum 2 gepresst, der mit dem flüssigen Knochenzement 4 vom distalen Ende des Schaftes 3 aus gefüllt wird, wobei - wie erwähnt - ein Abfliessen von Knochenzement 4 in das Knochenmark 6 hinein durch die Sperre 12 verhindert wird.

Ist der Hohlraum 2 - der in der Resektionsebene des Schenkelhalses gegebenenfalls beispielsweise durch eine Abdeckplatte abgedeckt, sein kann, um eine Verdichtung des Zementes 4 zu ermöglichen, - mit Knochenzement 4 gefüllt, so wird der Spritzenzylinder 16 entfernt. Solange der Knochenzement noch nicht ausgehärtet ist, besteht noch zusätzlich die Möglichkeit 10 proximal durch einen Deckel, in diesem Fall eine einfache Schraube, zu verschliessen, was nicht ausdrücklich dargestellt ist. Unvermeidbar im Gewinde 15 vorhandene Zementreste können dabei für eine solche Schraube nach ihrem Aushärten eine Sicherung gegen unbeabsichtigtes Lösen bilden.

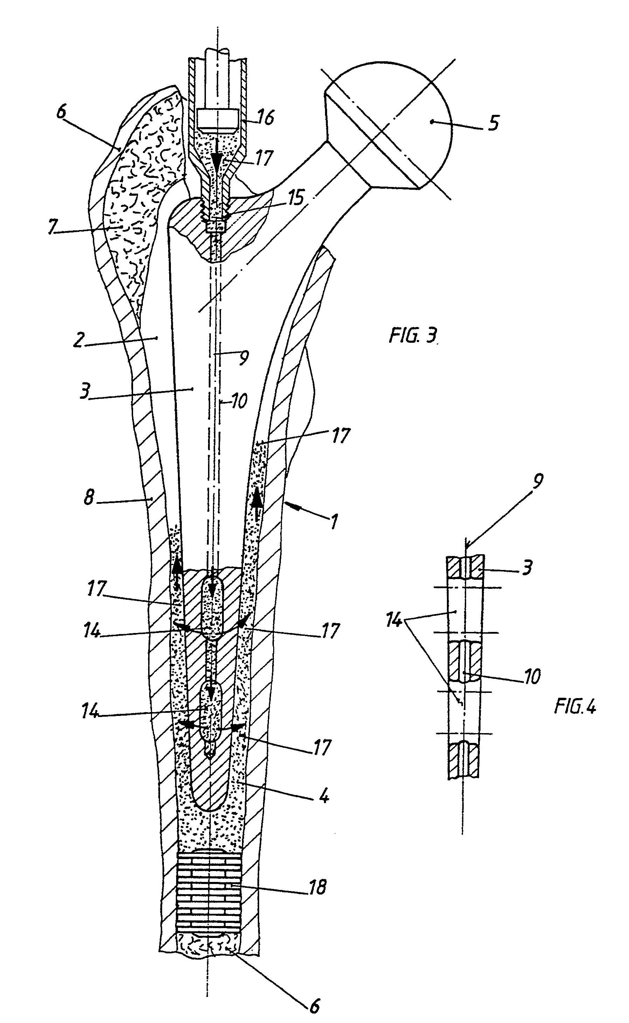

Das Ausführungsbeispiel nach den Fig. 3 und 4 unterscheidet sich von demjenigen nach Fig. 1 und 2 lediglich dadurch, dass die Bohrung 10 im Schaft 3 als Sackbohrung ausgebildet ist. Die im ersten Beispiel das distale Ende der Bohrung 10 verschliessende Markraumsperre 12 ist dabei durch einen vom Schaft 3 getrennten Sperrkörper 18 ersetzt, der vor dem Einsetzen des Schaftes 3 und dem Einfüllen des Knochenzementes 4 als distaler Abschluss in den Hohlraum 2 eingesetzt ist. Weiterhin sind im zweiten Beispiel zwei in Richtung der Längsachse 9 hintereinander angeordnete Durchbrüche 14 vorgesehen.

## Ansprüche

1. Verankerungsschaft für eine Femurkopfprothese (5), der mit Hilfe eines Knochenzementbettes (4) im Femur (1) fixiert werden soll, wobei der Schaft (3) in Richtung seiner zentralen Längsachse (9) eine Bohrung (10) aufweist, die am distalen Ende verschlossen ist, dadurch **gekennzeichnet**, dass die Bohrung (10) über mindestens einen Durchbruch im distalen Bereich mit der Aussenseite des Schaftes (3) in Verbindung steht.

2. Verankerungsschaft nach Anspruch 1, dadurch gekennzeichnet, dass der Durchbruch (14) nach ventral/dorsal gerichtet ist.

3. Verankerungsschaft nach Anspruch 1 oder 2, dadurch gekennzeichnet, dass die Bohrung (10) den Schaft (3) auf seiner ganzen Länge durchsetzt und am distalen Ende durch eine eingesetzte Markraumsperre (12) verschlossen ist.

4. Verankerungsschaft nach einem der Ansprüche 1 bis 3, dadurch gekennzeichnet, dass das proximale Ende der Längsbohrung (10) zu einer Einsatzöffnung für ein mindestens näherungsweise druckdichtes Einsetzen einer Knochenzementspritze (16) ausgeformt ist.

5. Verankerungsschaft nach Anspruch 4, dadurch gekennzeichnet, dass die Einsatzöffnung ein Gewinde (15) zum Einschrauben der Spritze (16) trägt.

6. Verankerungsschaft nach Anspruch 4 oder 5, dadurch gekennzeichnet, dass das proximale Ende der Längsbohrung (10) durch einen Deckel, beispielsweise durch eine Schraube verschliessbar ist.

FIG. 1

FIG. 2

*FIG. 3*

*FIG. 4*

| | | |
|---|---|---|
| Europäisches Patentamt | **EUROPÄISCHER RECHERCHENBERICHT** | Nummer der Anmeldung<br><br>EP 90 81 0855 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 704 089 (FAHRER)<br>* Zusammenfassung; Abbildungen 1,2; Spalte 5, Zeilen 3-52 * | 1,2 | A 61 F    2/36 |
| Y | --- | 3-6 | |
| Y | EP-A-0 315 283 (YPMA)<br>* Abbildungen 3,6; Seite 5, Zeile 45 - Seite 6, Zeilen 1-20 * | 3 | |
| Y | US-A-4 274 163 (MALCOM)<br>* Zusammenfassung; Abbildungen * | 4-6 | |
| A | FR-A-2 622 791 (GARCIA)<br>* Abbildung 4; Seite 8, Zeilen 10-12 * | 6 | |
| A | US-A-4 653 487 (MAALE)<br>--- | | |
| A | GB-A-2 082 072 (BROWN)<br>--- | | |
| D,A | EP-A-0 331 623 (SULZER)<br>--- | | |
| E | US-A-4 888 022 (HUEBSCH)<br>* Ganzes Dokument *<br>--- | 1-6 | RECHERCHIERTE SACHGEBIETE (Int. Cl.5)<br><br>A 61 F |
| P,X | DE-A-3 937 786 (JANSSON)<br>* Ganzes Dokument *<br>----- | 1-5 | |

Der vorliegende Recherchenbericht wurde für alle Patentansprüche erstellt

| Recherchenort | Abschlußdatum der Recherche | Prüfer |
|---|---|---|
| DEN HAAG | 21-03-1991 | STEENBAKKER J. |